# EUROPEAN PATENT APPLICATION

(11) **EP 0 766 969 A1**
(43) Date of publication of application: **09.04.1997**
(21) Application number: 96500018.5
(22) Date of filing: 06.02.1996
(51) Int. Cl.: A61L 2/12, A61L 2/06, A61L 2/26

(54) **Pacifier boiling device for microwave oven**

(30) Priority: 24.08.1995 ES 9502232 U
(71) Applicant: PLASTICOS DE GALICIA, S.A., 36210 Vigo (Pontevedra) (ES)
(72) Inventor: Villar Otero, Domingo, 36210 Vigo (ES)
(74) Representative: De la Fuente Fernandez, Dionisio

(57) **Abstract**

Within a microwave oven the pacifier boiling device of the invention is placed for the sterilization of a pacifier internally contained, said pacifier being immersed in a given quantity of water contained in the interior of a main body (1), provided with a handle (2), and to the upper mouth of which a perimetrical skirt (4) of an internal body (3) has been fitted and adjusted, so maintaining the pacifier in position within the said main body and without any possibility of movement of the last toward the outside.

## Description

### OBJECT OF THE INVENTION

The present invention is referred to a device usable as a pacifier boiler in a microwave ovens, whose evident purpose is to achieve a complete sterilization of the pacifier, using the boiler introduced in the interior of a microwave oven, using a minimal quantity of water, and said sterilization being achieved in a sensibly small period of time.

### FIELD OF THE INVENTION

This invention has its application within the industry devoted to the manufacture of articles complementary to those of infantile application.

### DESCRIPTION OF THE INVENTION

The pacifier boiling device for microwave oven proposed by the invention, constitutes an evident novelty within the field of application of the same, since its utilization provides a device trained to accomplish the sterilization of a pacifier, by placing said device within a microwave oven.

In a more concrete way, the boiler device for pacifiers applicable in a microwave oven which is the object of the invention, is constituted from a main container adopting the general configuration of a jug, to which is incorporated a bored, second body and which is adjustable to the edge of the main container, to which is secured by means of a simple flexion.

The pacifier is located in the interior of the principal body and is put below the internal body, the first remaining located between both elements, and then being accomplished the addition of a small quantity of water to be submitted thereinafter to the performance of the microwave oven.

The pacifier is unable to float when the poured of the water that it has been submitted previously to boiling is effected, consequently being achieved a complete sterilization using a reduced quantity of water, and investing in this operation a minimal quantity of time.

### BRIEF DESCRIPTION OF THE DRAWINGS

In order to complement the description that is being accomplished and with the purpose of helping for a better understandlng of the characteristics of the invention, a sheet of drawings is accompanied to the present disclosure, as an integrating part of the same, in order to show with an illustrative and not limitative character, the following:

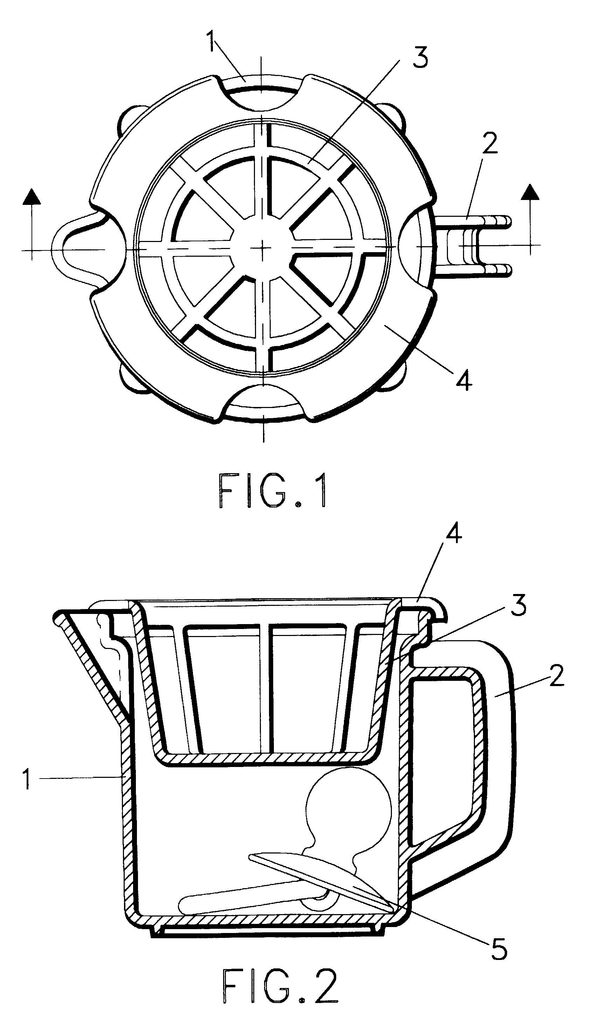

The Figure number 1.- Shows a top plan view of the object of the invention, consisting of a pacifier boiler for a microwave oven.

The Figure number 2.- Corresponds to a duly cross-sectioned elevational side view, of the object represented in the Figure number 1.

### PREFERRED EMBODIMENT OF THE INVENTION

At the sight of these Figures, it can be observed that the pacifier boiling device for microwave oven is constituted as a main container (1) adopting a jug-shape, provided with a suitable seized means (2) or handle, and with a pouring peak (not referenced), to which a bored interior body (3) is adjusted, which is provided with a perimetrical sidewalk skirt (4) on its upper side that is fitted to the upper edge of the main container (1).

The pacifier is located within the main container (1), then being added the relevant water, so that the ensemble formed by the pacifier contained in the interior of the main container (1), the internal container (3) and the water, being submitted to the action of the microwave oven, so that once the sterilizer function has been accomplished this ensemble can be removed from the interior of the microwave oven, the poured of the water contained in the interior of the recipient (1) being then accomplished through its pouring peak, without floating at any time the pacifier located between both bodies and said pacifier having been totally sterilized in a substantially reduced term of time with the use of a minimal quantity of water.

It is not considered necessary to make vaster the content of this description so that any expert in the art may understand the scope of the invention and the advantages derived from the same.

The materials, shape, size and arrangement of the elements will be susceptible of variation provided that this does not suppose an alteration in the scope of the invention.

The terms in which this invention has been described will have to be taken always in a wide and not limitative sens.

## Claims

1. Pacifier boiling device for microwave oven, characterized in that it is constituted from a container (1) adopting the general configuration of a jug, provided with a handle (2) and with a pouring peak, in which interior a bored body (3) is fitted to the first, and being provided with a perimetrical sidewalk skirt (4) which is adjusted to the mouth of the recipient (1).

2. Pacifier boiling device for microwave oven, according to the claim 1, characterized in that the pacifier (5) remains located in the interior of the main body (1), immersed in a given quantity of water and blocked toward the outside by means of the internal body (3).
